# EUROPEAN PATENT SPECIFICATION

(11) **EP 3 086 631 B1**
(45) Date of publication and mention of the grant of the patent: **18.10.2017**
(21) Application number: 14830886.9
(22) Date of filing: 18.12.2014
(51) Int. Cl.: A01C 11/02, A01G 9/08, A01H 4/00, A01C 5/04

(54) **PLANTING METHOD AND DEVICE FOR PLANT PROPAGULES**
PFLANZVERFAHREN UND VORRICHTUNG FÜR PFLANZENDIASPOREN
PROCÉDÉ ET DISPOSITIF DE PLANTATION POUR PROPAGULES VÉGÉTALES

(30) Priority: 27.12.2013 US 201361921234 P; 30.01.2014 SE 1450091
(43) Date of publication of application: 02.11.2016
(73) Proprietor: Georgia Tech Research Corporation, Atlanta, Georgia 30332-0415 (US)
(72) Inventor: AIDUN, Cyrus K., Marietta, Georgia 30067 (US)
(74) Representative: Novitas Patent AB
(86) International application number: PCT/IB2014/067084
(87) International publication number: WO 2015/097603

(56) References cited:
- WO-A2-2009/088774
- US-A- 4 323 019
- US-A1- 2008 078 117

## Description

### FIELD OF THE INVENTION

The invention relates to the field of planting plant propagules, in particular to tools and methods for planting.

### BACKGROUND TO THE INVENTION

The use of cuttings is a well-established method for propagation of plants, and it is well known that some plants are easier to propagate than others. In forestry replantation for example, there is need for large numbers of trees with good growth properties. Eucalyptus (Eucalyptus sp.) and Poplar (Populus sp.) are two examples of trees which today are propagated by cuttings. Other trees, like conifers such as pine, spruce and lark of different species are more difficult or impossible to propagate by cutting. An alternative method for large scale propagation of plants difficult to propagate with cuttings is to use somatic embryogenesis.

Cuttings generally lack a root when they are planted. Somatic embryos develop a root at a late stage and when transferred to a proper light and medium the shoot will develop.

Several methods and devices has been used and developed for planting small plants.

In the patent US 5,142,814 a planting device is presented where small plants with well-developed roots within a plug of soil are transported in water and then planted in soil in a tray. The device as presented can handle also multiple plants.

In the patent US 5,573,558 a similar device and method is presented for planting plants with well-developed roots, the in this patent the rooted plants are dragged by a vacuum through a tube down to the soil where it should be planted. None of them provide a method or a device that can set plant propagules in a substrate. A further device of this kind is known from WO 2009/088774 A2.

A problem with planting small plants, germinating somatic embryos or cuttings with a limited developed root is the weakness of the root. These is a high risk that the root of the plant propagule might be damaged when planted using a forceps to hold the root and place it in the substrate, whereby the fraction of viable plantlets may be low.

There is also need for planting tools that can be used in automated processes, in order to increase the throughput of planted propagules.

It is an object of the present invention to provide an improved or at least alternative solution for planting plant propagules.

### BRIEF DESCRIPTION OF THE DRAWINGS

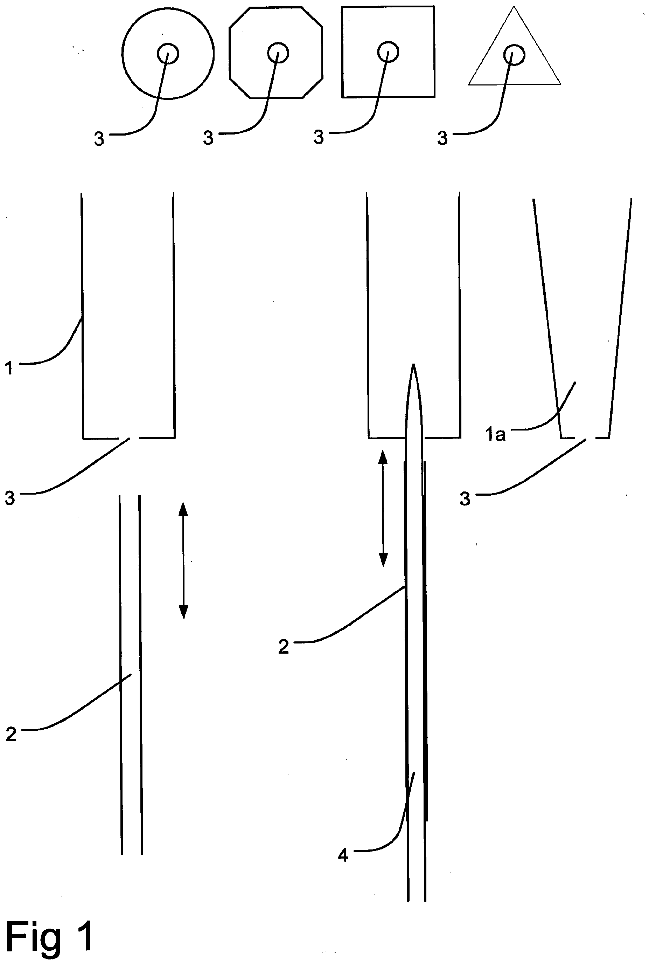

Figure 1, depicts a planting chamber (1), a tubular hollow member (2), a hole at the bottom (3) of the planting chamber, and a rod-like capping element (4) exemplified by a rod that fits inside the hollow member having a conical tip (2). The double headed arrow indicates that the member and the capping element can be moved in two directions. They can also be moved independently of each other. Alternative shapes for the planting chamber are also illustrated.
Figure 2 illustrates a planting method of the invention:
   A. A hollow member (2) with a capping element (4) is provided inside a planting chamber (1) filled with a growth substrate.
   B. A rooted plant, plant propagule or a rooted somatic embryo is placed inside the hollow member (2). The arrow (5) indicates the way air is flowing due to suction provided.
   C. The hollow member is withdrawn through the growth substrate in the direction opposite of the shoot forming end leaving the rooted plant inside the growth substrate.
   D. The rooted plant, plant propagule or the rooted somatic embryo shown properly planted in the substrate.

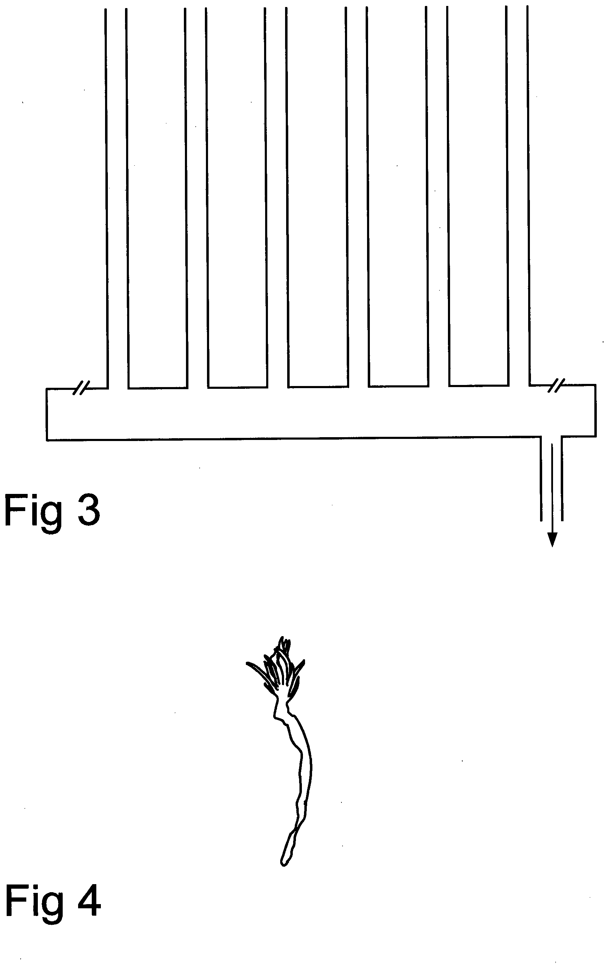

Figure 3: Shows one example of how a set of hollow members can be arranged such that they can be maneuvered in unison, and such that one outlet can be used to provide suction to all the hollow members.
Figure 4: Examples of plant propagules

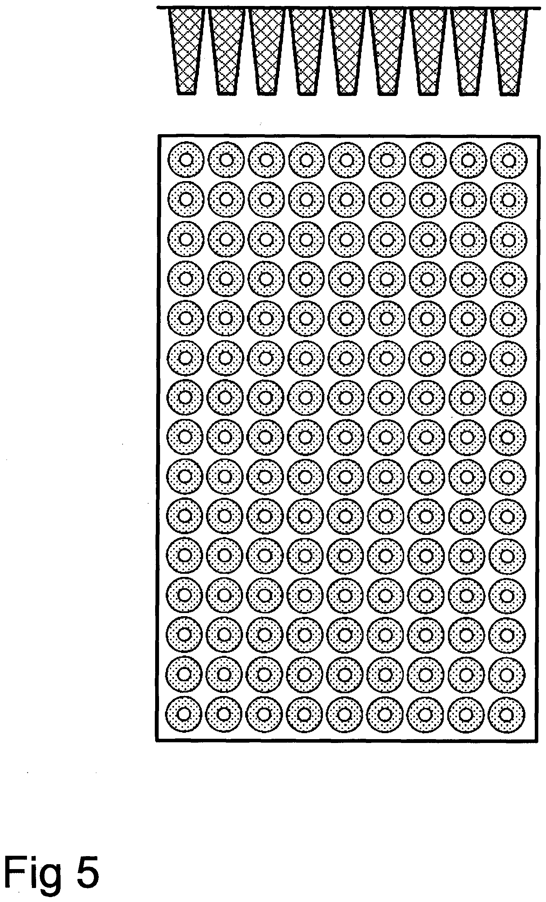

Figure 5: Shows an example of a tray with 135 pots.

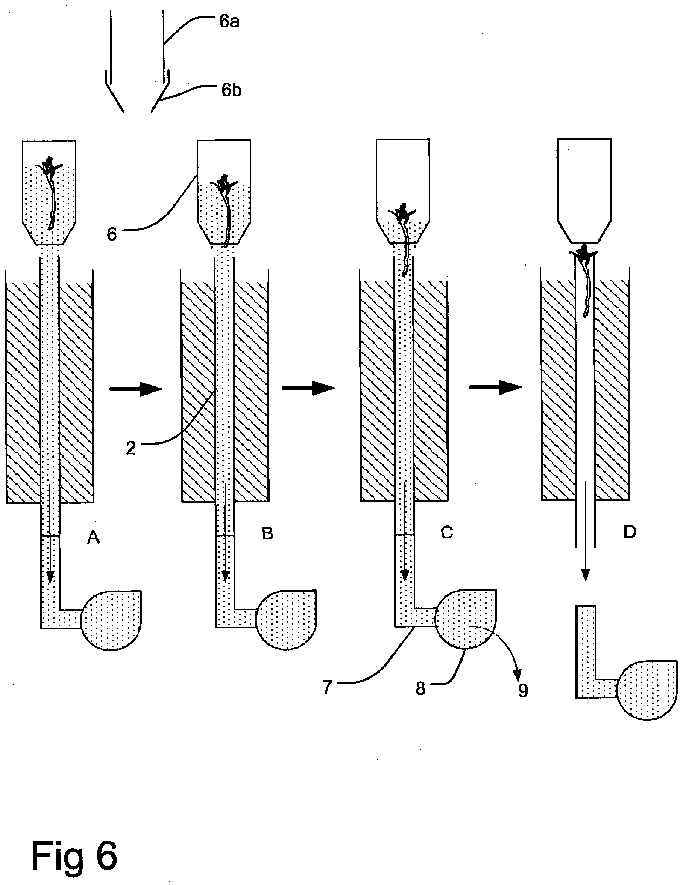

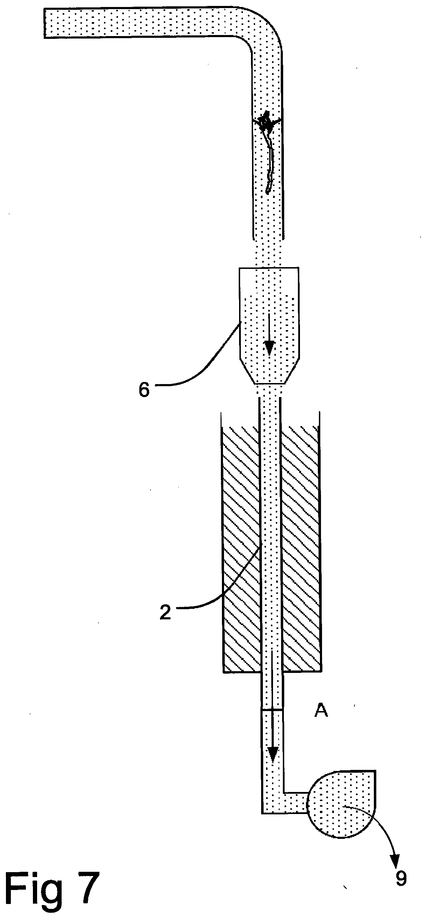

Figure 6. A schematic drawing of a planting chamber in the automatic design, and a planting process where the propagule is carried in liquid.

### SUMMARY OF THE INVENTION

The object of the invention is met with a method according to claim 1 and a device according to claim 10.

### DEFINITIONS

The term "plant propagule" refers to a plant, a part of a plant or a vegetative part of a plant with a root forming end and a shoot forming end. A plant propagule can be grown outdoors, indoors or cultured in vitro. Plant propagule includes plants germinated from seeds, somatic embryos and *in vitro* grown shoots from calluses or cuttings, but not the seed itself.

The term "root forming end" refers to the end of a propagule that will form a root when placed in a suitable substrate.

The term "shoot forming end" refers to the end of a propagule with a shoot apical meristem (SAM), which may develop into buds, leaves or cotyledons. The cotyledon is the shoot forming end of somatic embryos. In the present invention the shoot forming end of the plant propagule to be planted may preferably have a geometric shape such that it does not pass through the hollow member, i.e. the shoot may be at least in some dimensions be wider than the root forming end and larger than the cross-section of the hollow member. Preferably, the width of the shoot forming end should be at least 1.5 times the width of the root forming end.

The term "cuttings" refers to any part of a plant that will form roots when placed in appropriate substrate.

The term "planting chamber" refers to a cavity or vessel with an open top and a semi closed bottom that can hold substrate. An example of a chamber is a pot. Several chambers or pots can be arranged an array in a grid, which can be called a tray. One or several pots can be filled with a substrate simultaneously. The chamber can be made of for example metal, clay, wood or a plastic material. The planting chambers may have holes at the bottom to allow excess water to pass through. In a preferred planting chamber there is a guide or hole (3) for the hollow member (2).

The term "hollow member" refers to a tubular element with a hollow interior. It can be made of any suitable material, such as ceramics, glass, plastic or metal. The dimensions of the hollow member are selected based on the plant propagule to be handled, following the teachings herein below.

The term "growth substrate" refers to a material in which a plant propagule can root and grow, such as peat moss, soil, solid or semisolid medium.

### DETAILED DESCRIPTION OF THE INVENTION

The present invention provides methods and devices for planting plant propagules. Advantages of the invention include the capability of planting a plant propagule effectively, consistently and precisely at the centre of a planting chamber. A particular advantage is amenability for handling multiple propagules in an industrial manner. Additionally, the shoot is consistently placed over the surface of the growth substrate. Furthermore, the delicate root and shoot are handled gently.

### Method for planting a plant propagule

The present invention provides a method for planting a plant propagule with a root forming end and a shoot forming end.

### Hollow member and a growth substrate

Said method comprises the step (a) of providing a growth substrate and an elongated tubular hollow member. The hollow member may at its simplest be a round, straight tube of suitable dimensions (see below), but other suitable forms are also contemplated. The hollow member may for example have circular, oval, square, rectangular or triangular cross-section. The cross-section may be symmetrical, non-symmetrical or even irregular. Preferably, the cross-section of the hollow member is substantially constant, most preferably the cross section is constant. The hollow member may be a tube with circular cross-section having diameter of 2-8 mm, preferably 3-6 mm, most preferably 3-4 mm, having walls with thickness of less than 1 mm, preferably 0.1-0.6 mm, more preferably 0.2-0.5 mm, most preferably 0.3-0.4 mm.

The hollow member may be straight or curved, as long as it is suitable for use in the method. A straight hollow member is preferable. The length of the hollow member is suitably dimensioned such that the hollow member is longer than the depth of the substrate in which the propagule is to be planted. The length may be 5-50 cm, preferably 10-30 cm, more preferably 15-25 cm, most preferably 18-22 cm.

The inside of the hollow member should be smooth and should not contain any features that would result in fastening of a propagule within the hollow member or that might damage the propagule. The hollow member can be made of any suitable material, such as ceramics, glass, plastic or metal.

Irrespective of shape, the hollow member is dimensioned such that the root forming end of the propagule to be planted fits into the member. The root forming end must fit such that it does not get stuck.

For propagules containing a shoot forming end that is substantially larger than the root forming end the dimensions may be chosen such that when the root forming end is inserted in the member, the shoot forming end at least partially protrudes outside of the cross-section of the hollow member when viewed from the axial direction of the hollow member.

For propagules where the root forming end and the shoot forming end do not substantially differ in size, the dimension are chosen based on the root forming end, but preferably the walls of the hollow member are kept as thin as possible so as to minimize the hole made in the substrate.

It should be understood from the above that the exact dimensions and shapes, while important, are chosen based on the particular plant propagule to be planted, and may thus vary considerably. The skilled person will be able to choose the proper dimensions and shapes without undue burden, by simply measuring the typical root cross-sectional dimension and the typical shoot cross-sectional dimension, and then choosing a tubular hollow member fulfilling the criteria set forth above.

Said hollow member is arranged such that the hollow member is movable in relation to the substrate, and arranged such that the hollow member penetrates through the substrate. For example, the substrate might be in a planting chamber such as a pot, and the hollow member might be movably arranged in a hole in the bottom of the planting chamber, such that it extends thought the substrate. It is also envisioned that the growth substrate might be a solid piece of growth substrate, such as a peat block, in which case there would not be a requirement for a planting chamber. In such case, the hollow member would simply penetrate the growth substrate.

The hollow member may be arranged at the centre of a bottom wall of a planting chamber holding the growth substrate, to ensure consistent and accurate planting of the propagule at the center of the planting chamber.

Said step a) may optionally further comprise the step of providing a planting chamber filled with a growth substrate, followed by inserting the hollow member through the bottom wall of the chamber and through the growth substrate. This optional step may further comprise a second optional step that prior to the insertion, the hollow member is provided with a capping element placed inside the hollow member, and wherein the insertion is carried out with the capping element in place to prevent growth substrate from entering the hollow member, and wherein the capping element is removed prior to the insertion of the root forming end.

Alternatively, said step a) may optionally further comprise the step of providing a planting chamber filled with a growth substrate, followed by inserting the hollow member through the growth substrate in the direction of the bottom wall. This optional step may further comprise a second optional step that prior to the insertion, the hollow member is provided with a capping element capping the hollow member, and wherein the insertion is carried out with the capping element in place to prevent growth substrate from entering the hollow member. Preferably, the capping element is inserted through the bottom wall of the chamber and through the growth substrate, connected with the hollow member thus capping it, followed by insertion of the capped hollow member through the growth substrate in a direction reverse to the capping element insertion.

The second optional step (either variant) enables insertion of the hollow member through the substrate without risk of clogging the inside of the hollow member with substrate. The capping element can be made of any suitable material, such as ceramics, glass, plastic or metal. The capping element may be a rod-like element arranged inside the hollow member, and may have a conical tip. The capping element may be a straight metal rod with a conical tip with a circular cross-section, about 5 cm longer than the hollow member. The diameter of the capping element may be such that when arranged inside the hollow member, there is 0.1-0.5 mm space between the hollow member and the capping element. In cases where the method comprises inserting the hollow member through the growth substrate in the direction of the bottom wall, the capping element need not enter the hollow member in order to cap it. For instance, a rod (preferably with a conical tip) may be dimensioned suitably for connecting with the hollow member capping the end, without substantially entering the hollow member.

In another variation, the end of the hollow member being inserted into the growth substrate can be shaped in a conical manner or other suitable shapes such that upon insertion into the substrate the so shaped end of the hollow member substantially prevents the substrate from entering the hollow member. With this variation, a capping element may not be required any longer.

### Insertion of the propagule

The method also comprises the step (b) of inserting the root forming end of the propagule into the hollow member such that the shoot forming end does not enter the hollow member.

It should be understood, that the insertion of the root forming end of the propagule can take place before the hollow member is arranged such that the hollow member penetrates through the growth substrate. This is in particular applicable in methods where the hollow member is caused to penetrate the substrate from the direction of the shoot forming end. Alternatively, the insertion of the root forming end of the propagule can take place after the hollow member is already arranged such that the hollow member penetrates through the growth substrate.

Provided that the hollow member is properly dimensioned, the insertion of the root forming end into the hollow member requires minimal physical force i.e. can be done gently. Also provided that the hollow member is properly dimensioned, the minimal use of physical force conveniently provides that the shoot does not enter the hollow member.

The insertion step may comprise providing suction in the hollow member to insert the root forming end into the hollow member by way of suction. Suction has the advantage of providing gentle force to pull the root in place. Pushing the root may increase the risk of damaging the propagule. Additionally, suction may be used to clear the inside of the hollow member of any growth substrate the might have inadvertently entered the hollow member in the previous step. The suction may be provided by a pump connected to the hollow member.

The propagule may also be carried into the hollow member in a liquid such as water or aqueous solution. This may be especially advantageous in cases where the propagules are provided in a liquid, such as when industrially handling somatic embryos in an automated fashion. The method may comprise the step of filling the hollow member with liquid prior to insertion of the root forming end, followed by draining of the fluid to insert a root forming end of a propagule into the hollow member. The liquid may be both provided and withdrawn through the hollow member (see Figure 6).

A propagule suspended in a liquid may also be inserted in a free jet of liquid.

### Planting by withdrawal of the hollow member

The method further comprises the step (c) of withdrawing the hollow member through the growth substrate in the direction opposite of the shoot forming end. This can be accomplished by moving the hollow member, moving the growth substrate or both.

In case of planting propagules with shoot forming end being larger than the root forming end, the withdrawal will bring the shoot forming end in contact with the surface of the growth substrate. Since the root forming end is loosely within the hollow member, the contact between the growth substrate and the shoot forming end will result in the root forming end remaining in place while the hollow member is moved. This has the end result of the propagule being planted in the hole in the substrate left by the hollow member, with the root forming end located within the substrate and the shoot forming end at the surface of the substrate, e.g. at or near the top surface of the substrate. As a result, the shoot forming end may protrude at least partially or completely out of the surface.

In case of planting propagules where the root forming end and the shoot forming end do not substantially differ in size, the step (c) comprises holding the propagule in place in relation to the growth substrate while withdrawing the hollow member.

Subsequent to step (c) the method may optionally comprise the step of vibrating the growth substrate, whereby it is ensured that the hole left by the hollow member in the substrate is fully collapsed.

### Optional simultaneous planting of multiple propagules

The method is particularly advantageous in an industrial setting, where the method is multiplexed, i.e. the method may involve manipulation of multiple propagules simultaneously.

The method may be used with a plurality of propagules simultaneously, where the plurality of propagules are planted concomitantly in a plurality of growth substrates using a plurality of hollow members arranged in an array. In such cases, the method step c) may involve moving the array of hollow members in relation to the growth substrates.

Also, a plurality of propagules may be planted concomitantly in a plurality of growth substrates arranged in an array using a plurality of hollow members. In such cases, the method step c) may involve moving the array of growth substrates in relation to the hollow members.

### Types of propagules to be planted with the method of the invention

The method is generally applicable to many types of propagules. Particular types of propagules that may be planted using the method disclosed above may be selected from spruce somatic embryos, pine somatic embryos, poplar cuttings and eucalyptus cuttings.

### Planting unit

The present invention provides a planting unit for planting a plant propagule with a root forming end and a shoot forming end. The unit is suitable to be used with the method discussed above, and the above teachings concerning the properties (such as material, shape and dimensions) of the hollow member are fully applicable to the construction of the unit.

The unit comprises:
a) a planting chamber having an open top, and a bottom wall provided with a hole through the bottom wall;
b) an elongated tubular hollow member movably arranged in the hole such that it can be removed from the chamber through the bottom wall, wherein each hollow member is dimensioned such that the root forming end fits into the hollow member, and preferably further such that the shoot forming end does not fit into the hollow member, so that when the root forming end is inserted in the member, the shoot forming end at least partially protrudes outside of the cross-section of the hollow member when viewed from the axial direction of the hollow member; and
c) a capping element movably arranged to enable capping of the hollow member to prevent substrate from entering the hollow member.

The capping element may have a conical tip. The capping element may be a rod-like element arranged inside the hollow member. Alternatively, the tip of the hollow member could have a closable element or features capable of substantially closing the tip of the hollow member and thus functioning as the capping element.

The closable element at the tip of the hollow member could be in a conical shape with a small hole or no hole at the tip to function as a capping element. Another method to replace the capping element could be to press the end of the hollow tube such that to flatten and substantially close the tip. The capping element with a sharp end can be permanently installed to the end of the hollow member. All these alternative methods function more or less the same as to prevent growth substrate from entering a plugging the hollow member, as well as, allow easy insertion of the hollow member into the substrate without forcing part of the substrate out through the hole of the bottom wall of the planting chamber.

The planting unit may comprise means of holding the propagule in place in relation to the growth substrate (e.g. an electric or pneumatic or other types of actuating holding member).

The planting unit may also comprise means of filling the hollow member with fluid (e.g. water or aqueous buffer), preferably from the direction opposite of the end where the root is to be inserted. The planting unit may also comprise means of withdrawing fluid from the hollow member, preferably in the direction away from the end where the root is to be inserted. The means could be e.g. a pump, a gravitational source of fluid or the like.

The planting unit may further comprise means of providing the propagule to be planted suspended in a liquid to the hollow member. Such means may comprise a funnel, which may interface with the hollow member by way of a flexible member, an O-ring, or the like.

The planting unit may comprise a growth substrate.

The planting unit may comprise means for vibrating the growth substrate or the planting chambers.

### Planting array

The present invention provides a planting array for planting a plurality of plant propagules each comprising a root forming end and a shoot forming end. The array is suitable to be used with the method disclosed above, and the above teachings concerning the properties (such as shape and dimensions) of the hollow member are fully applicable to the construction of the array. The array may be arranged in a regular grid-like pattern.

The planting array comprises:
a) a plurality of planting chambers each having an open top, and a bottom wall provided with a hole through the bottom wall, fixed in an array;
b) a plurality of elongated hollow members movably arranged through the hole of each chamber, fixed to a first plate, such that the plurality of hollow members can be maneuvered in unison,
   wherein the hollow members are dimensioned such that the root forming ends fit into the members,
   and preferably further such that the shoot forming ends do not fit into the members, so that when the root forming ends are inserted in the members, the shoot forming ends at least partially protrude outside of the cross-sections of the hollow members when viewed from the axial directions of the hollow members.

The plurality of planting chambers may be arranged on a tray.

The planting array may comprise a plurality of capping elements, fixed to a second plate, such that plurality of capping elements can be maneuvered in unison, to enable capping of the hollow members. The capping elements may be rod-like and may have a conical tip.

The planting array may further comprise a plurality of rod-like elements arranged inside the hollow members, fixed to a second plate, such that plurality of rod-like elements can be maneuvered in unison. Each rod-like element may have a conical tip.

The planting array may comprise means of holding the propagules in place in relation to the growth substrates.

The planting array may also comprise means of filling the hollow members with fluid (e.g. water or aqueous buffer), preferably from the direction opposite of the end where the roots are to be inserted.

The planting array may further comprise means of providing the propagule to be planted suspended in a liquid to the hollow members. Such means may comprise a funnel or a plurality of funnels, which may interface with the hollow member by way of a flexible member, an O-ring, or the like.

The first plate may have means for providing suction to each of the hollow members.

The term "plate" used herein also encompasses other means having similar function, such as grids, scaffolds and the like.

The planting chambers may contain a growth substrate.

The planting array may comprise means for vibrating the growth substrate or the planting chambers.

### Integration with an automated process

The placement of the propagules may be done by hand as seen in Example 1, but an advantage with the invention is that the same methods and devices can be used without the need of human interference.

In a preferred setting the propagules are transported by a fluid, preferably comprising water, to the top of the hollow member, which is inside the planting chamber filled with the growth substrate. A nozzle (6) may be used to guide the propagule to the inlet of the hollow member. The bottom of the tube in an automated setting is attached or connected to means for providing suction, whereby a propagule will be sucked into the tube. Examples of means for providing suction is a pump (8) preferably attached to a tube (7), but gravity may also be used for removing the fluid.

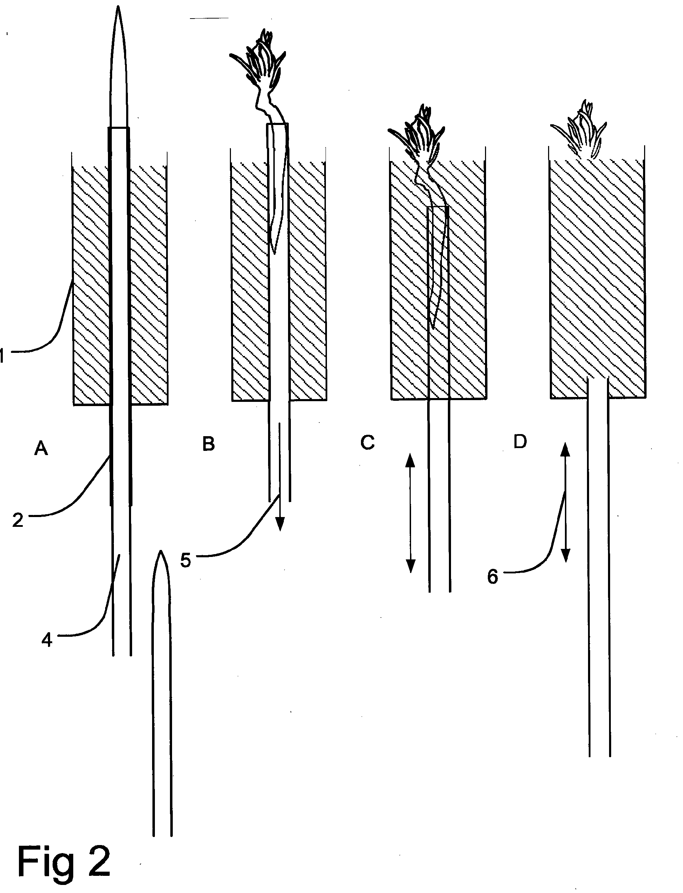

In figure 6, such process is exemplified; Fig 6A, a propagule has just entered a water filled nozzle, the pump (8) removes the water through the tube (7) and drag the propagule root first into the tube. As soon as the propagule has reached and stopped at the top of the hollow member (2) the water removing tube (7) at the bottom may be removed from the hollow member (2), which can be subsequently be removed from the chamber as seen in figure 2C and 2D. In the way the propagule will be planted without manual handling. The nozzle may be formed by a nozzle top (6a) and a nozzle bottom (6b). The nozzle bottom can be made of two or more separate parts. When brought together, they may form a tight junction (e.g. cone-shaped as illustrated in Fig 6) between the nozzle top (6a) and the hollow member (2).Other methods could be used to place the root of the propagule inside the hollow member before or after the hollow member is placed inside the substrate.

Any examples provided herein are not to be interpreted as limiting the scope of the invention.

Any references cited herein are hereby incorporated in their entirety.

### EXAMPLES

### Example 1:

A tray with 9 x 15 pots (135 pots) with diameter of 21 mm at the top and 13.5 mm at the bottom was filled with peat-moss growth substrate. In the filled tray, 135 germinated somatic embryos from Norwegian spruce were manually planted with a forceps.

A similar tray with 135 pots was filled with the same substrate. A planting array with 135 hollow members (tubes) with capping elements (needles) inside were allowed to penetrate the substrate creating a hole in each pot in the peat-moss substrate.

The hollow members were arranged to match the arrangement of the tray. The hollow members were straight metal tubes circular in cross-section were about 5 mm in outer diameter, about 4 mm in inner diameter.

The capping elements were metal needles with circular cross-section snugly fitting in the hollow members, and arranged in an array matching the arrangement of the hollow members.

After the removal of the needles, the 135 tubes were connected to a vacuum pump to provide suction, in order to aid placement of the germinated somatic embryos from Norwegian spruce into the tubes. When all germinated somatic embryos had been (manually) placed inside the hollow members, the hollow members were withdrawn by lifting the tray, leaving the root inside the substrate and the shoot on top of the substrate.

Both trays were incubated at 20 °C and watered 3 times a week. The plants were fertilized once a week. At given times the number of good plantlets was counted, table 1.

Surprisingly, it was noted that the trays planted with the planting device gave close to 80 % plantlets of excellent quality. The survival rate was 95 % or higher. The results were much improved compared to manual planting.

**Table 1:**

| Germinated Norwegian spruce somatic embryo | Number planted | Results | |
|---|---|---|---|
| | (n) | Excellent quality (n) | Survival (n) |
| Manually | 135 | 51 (33%) | 110 (81%) |
| Planted with the new device | 135 | 106 (78 %) | 128 (95 %) |

Similar results were obtained using poplar cuttings, data not shown.

### Example 2:

A similar tray with 9 x 15 pots (135 pots) as described in the previous example was prepared. In this setting, a single conical nozzle member with an opening at the bottom, and a fitting element substantially water-tightly connecting with the hollow members was used. For a fitting element, a short piece of flexible silicon tubing was used.

The hollow members and the conical nozzle member were filled with water by pumping from below (such that the hollow member was filled first, followed by the nozzle member). A germinated Norwegian spruce somatic embryo suspended in water was provided to the nozzle. The water was drained through the hollow member thus drawing the germinated somatic embryo into the hollow member.

The root forming end of the germinated somatic embryos entered the tube well, after which the tray was handled as in Example 1.

The number of plantlets of excellent quality was about the same as in Example 1.

### Example 3:

Trays with 9 x 15 pots (135 pots), as described in Example 1, were prepared. A device with actuation systems (Z-actuator) for lowering and lifting the trays filled with substrate was constructed to automatically lower the tray and insert the hollow members (metallic tubes) in the substrate. The metallic tubes were fitted (capped) with metallic rods with conical tips for easy penetration into the substrate. The metallic rods were arranged in an array to coincide with the centre of the pots. The tray with the substrate held by the Z-actuator was lowered onto the capped metallic tubes. The metallic tubes penetrated the substrate in the pots exposing the top of the tube with the caps. The 135 metallic rods were removed simultaneously by two parallel sliding plates equipped with a gripping mechanism exposing the open top of the metallic tubes ready for insertion of propagules. It is shown that with actuation systems, the method can be automated so to plant large trays with multiple pots simultaneously in very short time. These experiments were designed to demonstrate the potential of described method for automation in large scale operations.

### Example 4:

Over 80 similar trays with 9 x 15 pots (135 pots), as described in Example 1, were prepared. The hollow members were arranged to match the arrangement of the tray. The hollow members were straight metal tubes circular in cross-section were about 5 mm in outer diameter, about 4 mm in inner diameter.

The capping elements were metal needles with circular cross-section snugly fitting in the hollow members, and arranged in an array matching the arrangement of the hollow members.

In this case no suction was provided and the roots of germinated Norwegian spruce somatic embryos with proper shoots were placed directly into the hollow members. When all germinated somatic embryos had been placed inside the hollow members, the hollow members were withdrawn by lifting the tray, leaving the root inside the substrate and the shoot on top of the substrate.

The trays were incubated at 20°C and watered 3 times a week. The plants were fertilized once a week. It was shown that about 85% of the planted germinated somatic embryos with this method developed into viable plants with robust growth.

## Claims

1. A method for planting a plant propagule with a root forming end and a shoot forming end, comprising the steps of:
a) providing a growth substrate and an elongated tubular hollow member (2),
arranged such that the hollow member (2) is movable in relation to the substrate, and
arranged such that the hollow member (2) penetrates through the substrate, and
wherein said hollow member (2) is dimensioned such that the root forming end fits into the member (2);
b) inserting the root forming end of the propagule into the hollow member (2), either before or after that the hollow member (2) is arranged to penetrate through the substrate; **characterized in that** it further comprises the step of
c) withdrawing the hollow member (2) through the growth substrate in the direction opposite of the shoot forming end, such that the propagule is planted in the hole in the substrate left by the hollow member (2), with the root forming end located within the substrate and the shoot forming end located at the surface of the substrate.

2. The method according to claim 1, where
(i) the propagule has a shoot forming end substantially larger than the root forming end, wherein
the step a) comprises the provision of a hollow member (2) dimensioned such that when the root forming end is inserted in the member (2), the shoot forming end at least partially protrudes outside of the cross-section of the hollow member (2) when viewed from the axial direction of the hollow member (2), and
the step b) is performed such that the shoot forming end does not fully enter the hollow member (2); or
(ii) the propagule has a shoot forming end not substantially larger than the root forming end, wherein the step c) comprises holding the shoot forming end in place in relation to the substrate.

3. The method according to any of the preceding claims, wherein the step c) further comprises vibrating the substrate.

4. The method according to any of the preceding claims, wherein the growth substrate is provided arranged in a planting chamber (1) having a bottom wall, and the hollow member (2) is arranged such that it penetrates through said bottom wall.

5. The method according to any of the preceding claims, wherein
(i) step a) further comprises providing a planting chamber (1), inserting the hollow member (2) through the bottom wall of the chamber (1), and subsequently filling the chamber (1) with the growth substrate; or
(ii) step a) further comprises providing a planting chamber (1) having a bottom wall filled with a growth substrate, followed by inserting the hollow member (2) through the bottom wall of the chamber (1) and through the growth substrate.

6. The method according to claim 5 alternative (ii), wherein the step a) further comprises that prior to the insertion, the hollow member (2) is provided with a capping element (4) placed inside the hollow member (2), and wherein the insertion is carried out with the capping element (4) in place to prevent growth substrate from entering the hollow member (2), and wherein the capping element (4) is removed prior to the insertion of the root forming end.

7. The method according to any of claims 1-4, wherein step a) further comprises providing a planting chamber having a bottom wall filled with a growth substrate, followed by inserting the hollow member (2) through the growth substrate in the direction of the bottom wall of the chamber (1).

8. The method according to claim 7, wherein in step a) the hollow member (2) is provided with a capping element for capping the hollow member (2), and wherein the insertion is carried out with the capping element in place such as to prevent growth substrate from entering the hollow member (2).

9. The method according to claim 8, wherein the capping element is inserted through the bottom wall of the chamber (1) and through the growth substrate, connected with the hollow member (2) thus capping it, followed by insertion of the capped hollow member (2) through the growth substrate in a direction reverse to the capping element insertion.

10. A planting unit for planting a plant propagule with a root forming end and a shoot forming end, comprising:
a) a planting chamber (1) having an open top, and a bottom wall provided with a hole (3) through the bottom wall; **characterized in that** it further comprises
b) an elongated tubular hollow member (2) movably arranged in the hole (3) such that it can be removed from the chamber through the hole (3), wherein the hollow member (2) is dimensioned such that the root forming end fits into the hollow member (2); and
c) a capping element (4) movably arranged to enable capping of the hollow member (2).

11. The planting unit according to claim 10, wherein the hollow member (2) is dimensioned such that the shoot forming end does not fit into the hollow member (2), and such that when the root forming end is inserted in the member (2), the shoot forming end at least partially protrudes outside of the cross-section of the hollow member (2) when viewed from the axial direction of the hollow member (2).

12. The planting unit according to claim 10 or 11, wherein the planting unit further comprises means of holding the propagule in place in relation to the growth substrate and/or means for vibrating the planting chamber.

13. The planting unit according to any of claims 10-12, wherein the capping element (4) has a conical tip, and/or wherein the capping element (4) is a rod-like element arranged inside the hollow member (2).

14. A planting array for planting a plurality of plant propagules each comprising a root forming end and a shoot forming end, comprising
(i) a plurality of planting units according to any of claims 10-13 arranged in an array; or
(ii)
a) a plurality of planting chambers each having an open top, and a bottom wall provided with a hole through the bottom wall, fixed in an array; and
b) a plurality of elongated hollow members movably arranged through the hole of each chamber, fixed to a first plate, such that the plurality of hollow members can be maneuvered in unison,
wherein the hollow members are dimensioned such that the root forming ends fit into the members.

15. The planting array according to claim 14 alternative (ii), further comprising a plurality of capping elements, fixed to a second plate, such that plurality of capping elements can be maneuvered in unison, to enable capping of the hollow members.

## Patentansprüche

1. Verfahren zum Pflanzen einer Pflanzenfortpflanzungseinheit mit einem wurzelbildenden Ende und einem sproßbildenden Ende, das folgende Schritte umfasst:
a) Bereitstellen eines Wuchssubstrats und einen länglichen röhrenförmigen hohlen Elements (2), die derart angeordnet sind, dass das hohle Element (2) bezogen auf das Substrat beweglich ist, und
die derart angeordnet sind, dass das hohle Element (2) das Substrat durchdringt, und
wobei das hohle Element (2) so bemessen ist, dass das wurzelbildende Ende in das Element (2) passt;
b) Einführen des wurzelbildenden Endes der Fortpflanzungseinheit in das hohle Element (2), entweder bevor oder nachdem das hohle Element (2) so angeordnet ist, dass es das Substrat durchdringt;
**dadurch gekennzeichnet, dass** es ferner folgenden Schritt umfasst:
c) Herausziehen des hohlen Elements (2) durch das Wuchssubstrat in der Richtung entgegengesetzt zum sproßbildenden Ende, sodass die Fortpflanzungseinheit in das Loch im Substrat gepflanzt ist, das von dem hohlen Element (2) freigelassen wird, wobei sich das wurzelbildende Ende im Substrat befindet und sich das sproßbildende Ende an der Oberfläche des Substrats befindet.

2. Verfahren nach Anspruch 1, wobei
(i) die Fortpflanzungseinheit ein sproßbildendes Ende aufweist, das wesentlich größer ist als das wurzelbildende Ende,
wobei
Schritt a) das Bereitstellen eines hohlen Elements (2) umfasst, das so bemessen ist, dass wenn das wurzelbildende Ende in das Element (2) eingeführt wird, das sproßbildende Ende bei Betrachtung aus der axialen Richtung des hohlen Elements (2) zumindest teilweise aus dem Querschnitt des hohlen Elements (2) ragt, und
Schritt b) derart durchgeführt wird, dass das sproßbildende Ende nicht vollständig in das hohle Element (2) eindringt; oder
(ii) die Fortpflanzungseinheit ein sproßbildendes Ende aufweist, das nicht wesentlich größer ist als das wurzelbildende Ende, wobei Schritt c) ein Festhalten des sproßbildenden Endes bezogen auf das Substrat umfasst.

3. Verfahren nach einem der vorhergehenden Ansprüche, wobei Schritt c) ferner umfasst, dass das Substrat in Schwingungen versetzt wird.

4. Verfahren nach einem der vorhergehenden Ansprüche, wobei das Wuchssubstrat in einer Pflanzkammer (1) mit einer Bodenwand angeordnet vorgesehen wird und das hohle Element (2) so angeordnet wird, dass es die Bodenwand durchdringt.

5. Verfahren nach einem der vorhergehenden Ansprüche, wobei
(i) Schritt a) ferner ein Bereitstellen einer Pflanzkammer (1), ein Einführen des hohlen Elements (2) durch die Bodenwand der Kammer (1) und ein anschließendes Füllen der Kammer (1) mit dem Wuchssubstrat umfasst; oder
(ii) Schritt a) ferner ein Bereitstellen einer Pflanzkammer (1) mit einer Bodenwand, die mit einem Wuchssubstrats gefüllt ist, gefolgt von einem Einführen des hohlen Elements (2) durch die Bodenwand der Kammer (1) und durch das Wuchssubstrat umfasst.

6. Verfahren nach Anspruch 5 Alternative (ii), wobei Schritt a) ferner umfasst, dass das hohle Element (2) vor dem Einführen mit einem Verschlusselement (4) versehen wird, das in dem hohlen Element (2) platziert wird, und wobei das Einführen erfolgt, während sich das Verschlusselement (4) an seinem Platz befindet, damit verhindert wird, dass das Wuchssubstrat in das hohle Element (2) eindringt, und wobei das Verschlusselement (4) vor dem Einführen des wurzelbildenden Endes herausgenommen wird.

7. Verfahren nach einem der Ansprüche 1 bis 4, wobei Schritt a) ferner ein Bereitstellen einer Pflanzkammer mit einer Bodenwand, die mit einem Wuchssubstrat gefüllt ist, gefolgt von einem Einführen des hohlen Elements (2) durch das Wuchssubstrat in Richtung der Bodenwand der Kammer (1) umfasst.

8. Verfahren nach Anspruch 7, wobei in Schritt a) das hohle Element (2) mit einem Verschlusselement zum Verschließen des hohlen Elements (2) versehen wird, und wobei das Einführen erfolgt, während sich das Verschlusselement an seinem Platz befindet, damit verhindert wird, dass das Wuchssubstrat in das hohle Element (2) eindringt.

9. Verfahren nach Anspruch 8, wobei das Verschlusselement verbunden mit dem hohlen Element (2) durch die Bodenwand der Kammer (1) und durch das Wuchssubstrat eingeführt wird und es so verschließt, gefolgt von einem Einführen des verschlossenen hohlen Elements (2) durch das Wuchssubstrat in einer Richtung umgekehrt zum Einführen des Verschlusselements.

10. Pflanzeinheit zum Pflanzen einer Pflanzenfortpflanzungseinheit mit einem wurzelbildenden Ende und einem sproßbildenden Ende, umfassend:
a) eine Pflanzkammer (1) mit einem offenen Oberteil und einer Bodenwand, die mit einem Loch (3) durch die Bodenwand hindurch versehen ist; **dadurch gekennzeichnet, dass** sie ferner Folgendes umfasst:
b) ein längliches röhrenförmiges hohles Element (2), das derart beweglich in dem Loch (3) angeordnet ist, dass es durch das Loch (3) aus der Kammer herausgenommen werden kann, wobei das hohle Element (2) so bemessen ist, dass das wurzelbildende Ende in das hohle Element (2) passt; und
c) ein Verschlusselement (4), das beweglich angeordnet ist, damit ein Verschließen des hohlen Elements (2) ermöglicht wird.

11. Pflanzeinheit nach Anspruch 10, wobei das hohle Element (2) derart bemessen ist, dass das sproßbildende Ende nicht in das hohle Element (2) passt, und derart, dass wenn das wurzelbildende Ende in das Element (2) eingeführt wird, das sproßbildende Ende bei Betrachtung aus der axialen Richtung des hohlen Elements (2) zumindest teilweise aus dem Querschnitt des hohlen Elements (2) ragt.

12. Pflanzeinheit nach Anspruch 10 oder 11, wobei die Pflanzeinheit ferner Mittel zum Festhalten der Fortpflanzungseinheit bezogen auf das Wuchssubstrat und/oder Mittel zum Versetzen der Pflanzkammer in Schwingungen umfasst.

13. Pflanzeinheit nach einem der Ansprüche 10 bis 12, wobei das Verschlusselement (4) eine konische Spitze aufweist und/oder wobei das Verschlusselement (4) ein stabartiges Element ist, das in dem hohlen Element (2) angeordnet ist.

14. Pflanzanordnung zum Pflanzen von einer Vielzahl von Pflanzenfortpflanzungseinheiten, die jeweils ein wurzelbildendes Ende und ein sproßbildendes Ende umfassen, umfassend:
(i) eine Vielzahl von Pflanzeinheiten nach einem der Ansprüche 10 bis 13, die in einer Anordnung angeordnet sind; oder
(ii)
a) eine Vielzahl von in einer Anordnung fixierten Pflanzkammern, die jeweils ein offenes Oberteil und eine Bodenwand aufweisen, die mit einem Loch durch die Bodenwand hindurch versehen sind; und
b) eine Vielzahl von beweglich durch das Loch jeder Kammer angeordneten, länglichen hohlen Elementen, die derart an einer ersten Platte befestigt sind, dass die Vielzahl hohler Elemente gleichzeitig gemeinsam bewegt werden kann,
wobei die hohlen Elemente derart bemessen sind, dass die wurzelbildenden Enden in die Elemente passen.

15. Pflanzanordnung nach Anspruch 14 Alternative (ii), die ferner eine Vielzahl von Verschlusselementen umfasst, die an einer zweiten Platte befestigt sind, sodass die Vielzahl von Verschlusselementen gleichzeitig gemeinsam bewegt werden kann, damit die hohlen Elemente verschlossen werden können.

## Revendications

1. Procédé de plantation d'une propagule végétale dotée d'une extrémité formant racine et d'une extrémité formant pousse, comprenant les étapes consistant à :
a) mettre à disposition un substrat de croissance et un organe creux (2) tubulaire allongé, agencés de telle manière que l'organe creux (2) est mobile par rapport au substrat, et
agencés de telle manière que l'organe creux (2) pénètre à travers le substrat, et ledit organe creux (2) étant dimensionné de telle sorte que l'extrémité formant racine rentre dans l'organe (2) ;
b) insérer l'extrémité formant racine de la propagule dans l'organe creux (2), soit avant soit après que l'organe creux (2) est agencé pour pénétrer à travers le substrat ; **caractérisé en ce qu'**il comprend en outre l'étape consistant à
c) retirer l'organe creux (2) à travers le substrat de croissance dans la direction opposée à l'extrémité formant pousse, de telle sorte que la propagule est plantée dans le trou dans le substrat laissé par l'organe creux (2), l'extrémité formant racine étant située dans le substrat et l'extrémité formant pousse étant située à la surface du substrat.

2. Procédé selon la revendication 1, dans lequel
(i) la propagule comporte une extrémité formant pousse sensiblement plus grande que l'extrémité formant racine,
dans lequel
l'étape a) comprend la mise à disposition d'un organe creux (2) dimensionné de telle sorte que, quand l'extrémité formant racine est insérée dans l'organe (2), l'extrémité formant pousse dépasse au moins partiellement hors de la section transversale de l'organe creux (2), vu depuis la direction axiale de l'organe creux (2), et
l'étape b) est effectuée de telle sorte que l'extrémité formant pousse n'entre pas entièrement dans l'organe creux (2) ; ou
(ii) la propagule comporte une extrémité formant pousse qui n'est pas sensiblement plus grande que l'extrémité formant racine, l'étape c) comprenant le maintien en place de l'extrémité formant pousse par rapport au substrat.

3. Procédé selon l'une quelconque des revendications précédentes, dans lequel l'étape c) comprend en outre la vibration du substrat.

4. Procédé selon l'une quelconque des revendications précédentes, dans lequel le substrat de croissance est prévu agencé dans un compartiment de plantation (1) comportant une paroi de fond, et l'organe creux (2) est agencé de telle sorte qu'il pénètre à travers ladite paroi de fond.

5. Procédé selon l'une quelconque des revendications précédentes, dans lequel
(i) l'étape a) comprend en outre la mise à disposition d'un compartiment de plantation (1), l'insertion de l'organe creux (2) à travers la paroi de fond du compartiment (1), et ensuite le remplissage du compartiment (1) avec le substrat de croissance ; ou
(ii) l'étape a) comprend en outre la mise à disposition d'un compartiment de plantation (1) comportant une paroi de fond, rempli d'un substrat de croissance, suivie de l'insertion de l'organe creux (2) à travers la paroi de fond du compartiment (1) et à travers le substrat de croissance.

6. Procédé selon la revendication 5, alternative (ii), dans lequel l'étape a) comprend en outre qu'avant l'insertion, l'organe creux (2) est pourvu d'un élément de bouchage (4) placé à l'intérieur de l'organe creux (2), et dans lequel l'insertion est effectuée avec l'élément de bouchage (4) en place afin d'empêcher que du substrat de croissance entre dans l'organe creux (2), et dans lequel l'élément de bouchage (4) est retiré avant l'insertion de l'extrémité formant racine.

7. Procédé selon l'une quelconque des revendications 1 à 4, dans lequel l'étape a) comprend en outre la mise à disposition d'un compartiment de plantation comportant une paroi de fond, rempli d'un substrat de croissance, suivie de l'insertion de l'organe creux (2) à travers le substrat de croissance dans la direction de la paroi de fond du compartiment (1).

8. Procédé selon la revendication 7, dans lequel dans l'étape a), l'organe creux (2) est pourvu d'un élément de bouchage pour boucher l'organe creux (2), et dans lequel l'insertion est effectuée avec l'élément de bouchage en place de façon à empêcher que du substrat de croissance entre dans l'organe creux (2).

9. Procédé selon la revendication 8, dans lequel l'élément de bouchage est inséré à travers la paroi de fond du compartiment (1) et à travers le substrat de croissance, relié à l'organe creux (2), le bouchant ainsi, suivi de l'insertion de l'organe creux (2) bouché à travers le substrat de croissance dans une direction inverse à l'insertion de l'élément de bouchage.

10. Unité de plantation destiné à la plantation d'une propagule végétale dotée d'une extrémité formant racine et d'une extrémité formant pousse, comprenant :
a) un compartiment de plantation (1) comportant une partie supérieure ouverte, et une paroi de fond pourvue d'un trou (3) à travers la paroi de fond ; **caractérisée en ce qu'**elle comprend en outre
b) un organe creux (2) tubulaire allongé, agencé mobile dans le trou (3), de telle sorte qu'il peut être retiré du compartiment à travers le trou (3), l'organe creux (2) étant dimensionné de telle sorte que l'extrémité formant racine rentre dans l'organe creux (2) ; et
c) un élément de bouchage (4) agencé mobile pour permettre le bouchage de l'organe creux (2).

11. Unité de plantation selon la revendication 10, dans laquelle l'organe creux (2) est dimensionné de telle sorte que l'extrémité formant pousse ne rentre pas dans l'organe creux (2), et de telle sorte que, quand l'extrémité formant racine est insérée dans l'organe (2), l'extrémité formant pousse dépasse au moins partiellement hors de la section transversale de l'organe creux (2), vu depuis la direction axiale de l'organe creux (2).

12. Unité de plantation selon la revendication 10 ou 11, dans laquelle l'unité de plantation comprend en outre des moyens destinés à maintenir la propagule en place par rapport au substrat de croissance et/ou des moyens destinés à faire vibrer le compartiment de plantation.

13. Unité de plantation selon l'une quelconque des revendications 10 à 12, dans laquelle l'élément de bouchage (4) comporte un bout conique, et/ou dans lequel l'élément de bouchage (4) est un élément de type tige agencé à l'intérieur de l'organe creux (2).

14. Agencement de plantation destiné à la plantation d'une pluralité de propagules végétales comprenant chacune une extrémité formant racine et une extrémité formant pousse, comprenant
(i) une pluralité d'unités de plantation selon l'une quelconque des revendications 10 à 13 agencées en un agencement ; ou
(ii)
a) une pluralité de compartiments de plantation comportant chacun une partie supérieure ouverte, et une paroi de fond pourvue d'un trou à travers la paroi de fond, fixés en un agencement ; et
b) une pluralité d'organes creux allongés agencés mobiles à travers le trou de chaque compartiment, fixés à une première plaque, de telle sorte que la pluralité d'organes creux peut être manoeuvrée à l'unisson,
lesdits organes creux étant dimensionnés de telle sorte que les extrémités formant racine rentrent dans les organes.

15. Agencement de plantation selon la revendication 14, alternative (ii), comprenant en outre une pluralité d'éléments de bouchage, fixés à une seconde plaque, de telle sorte que la pluralité d'éléments de bouchage peut être manoeuvrée à l'unisson, afin de permettre le bouchage des organes creux.
